# EUROPEAN PATENT APPLICATION

(11) **EP 4 434 575 A1**
(43) Date of publication of application: **25.09.2024**
(21) Application number: 24164255.2
(22) Date of filing: 18.03.2024
(51) Int. Cl.: A61N 2/02

(54) **CANCER TREATMENT APPARATUS AND METHOD OF CONTROLLING CANCER TREATMENT APPARATUS**

(30) Priority: 23.03.2023 JP 2023046642
(71) Applicant: Ricoh Company, Ltd., Tokyo 143-8555 (JP); Public University Corporation Yokohama City University, Yokohama-shi, Kanagawa 236-0027 (JP)
(72) Inventor: Kishi, Kazuhito, Tokyo, 143-8555 (JP); Yamaguchi, Takashi, Tokyo, 143-8555 (JP); Hasegawa, Motokazu, Tokyo, 143-8555 (JP); Umemura, Masanari, Kanagawa, 236-0027 (JP); Ishikawa, Yoshihiro, Kanagawa, 236-0027 (JP)
(74) Representative: Marks & Clerk LLP

(57) **Abstract**

An alternating magnetic field having a frequency, at which a sufficient antitumor effect can be obtained, is enabled to be applied to a living body (P) for a previously prescribed period of time while restricting an amount of heat generation from the living body. A cancer treatment apparatus (100) includes a magnetic field generator (50) configured to generate an alternating magnetic field to be applied to a living body (P), and a magnetic field controller (22) configured to increase an intensity of the alternating magnetic field generated by the magnetic field generator (50) stepwise until a first period of time elapses since application of the alternating magnetic field to the living body, and to set the intensity of the alternating magnetic field to a previously set maximum value from elapse of the first period of time until a timing to end the application of the alternating magnetic field.

## Description

### BACKGROUND OF THE INVENTION

### Field of the Invention

The present disclosure relates to a cancer treatment apparatus and a method of controlling a cancer treatment apparatus.

### Description of the Related Art

Hyperthermia apparatuses including a ferromagnetic ferrite particle-containing ceramic heating element to be embedded near an in-vivo diseased site, and configured to put the diseased site within an alternating magnetic field to be generated from a coil, to thereby induce heat generation from the diseased site, and heat and treat the diseased site have been known (for example, see Japanese Unexamined Patent Application Publication No. 02-88059).

Cancer treatment apparatuses configured to apply an alternating magnetic field in a frequency range, in which a cancer cell proliferation inhibiting effect can be obtained, to a diseased tissue from a magnetic field generator that is deployed to surround a living body have been known (for example, see International Publication No. WO 2018/097185).

### SUMMARY OF THE INVENTION

According to an embodiment of the present disclosure, a cancer treatment apparatus includes a magnetic field generator configured to generate an alternating magnetic field to be applied to a living body, and a magnetic field controller configured to increase an intensity of the alternating magnetic field generated by the magnetic field generator stepwise until a first period of time elapses since application of the alternating magnetic field to the living body, and to set the intensity of the alternating magnetic field to a previously set maximum value from elapse of the first period of time until a timing to end the application of the alternating magnetic field. The cancer treatment apparatus according to an embodiment of the present disclosure is not based on the hyperthermic effect like a hyperthermia apparatus, but utilizes the antitumor effect of a magnetic field itself as in International Publication No. WO 2018/097185.

### BRIEF DESCRIPTION OF THE DRAWINGS

FIG. 1 is a view illustrating an example of the configuration of a cancer treatment apparatus according to a first embodiment;
FIG. 2 is a front view illustrating an overview of a magnetic field generator of FIG. 1;
FIG. 3 is a top view illustrating an overview of the magnetic field generator of FIG. 1;
FIG. 4 is a cross-sectional view of the magnetic field generator along a line Z1-Z2 in FIG. 3;
FIG. 5 is a drawing illustrating examples of waveforms of alternating magnetic fields applied to a patient by the cancer treatment apparatus of FIG. 1;
FIG. 6 is a graph illustrating an example of a relationship between the intensity of an alternating magnetic field generated from a coil of FIG. 1 and the body temperature of a patient;
FIG. 7 is a graph illustrating another example of a relationship between the intensity of an alternating magnetic field generated from the coil of FIG. 1 and the body temperature of a patient;
FIG. 8 is a graph illustrating still another example of a relationship between the intensity of an alternating magnetic field generated from the coil of FIG. 1 and the body temperature of a patient;
FIG. 9 is a graph illustrating another example of a relationship between the intensity of an alternating magnetic field generated from the coil of FIG. 1 and the body temperature of a patient;
FIG. 10 is a graph illustrating another example of a relationship between the intensity of an alternating magnetic field generated from the coil of FIG. 1 and the body temperature of a patient;
FIG. 11 is a graph illustrating another example in which the intensity of an alternating magnetic field generated from the coil of FIG. 1 is changed during a treatment time;
FIG. 12 is a view illustrating an example of the configuration of a cancer treatment apparatus according to a second embodiment;
FIG. 13 is a view illustrating another example of the magnetic field generator mounted on the cancer treatment apparatuses of FIG. 1 and FIG. 12;
FIG. 14 is a view illustrating still another example of the magnetic field generator mounted on the cancer treatment apparatuses of FIG. 1 and FIG. 12;
FIG. 15 is a view illustrating another example of the magnetic field generator mounted on the cancer treatment apparatuses of FIG. 1 and FIG. 12;
FIG. 16 is a view illustrating another example of the magnetic field generator mounted on the cancer treatment apparatuses of FIG. 1 and FIG. 12;
FIG. 17 is a view illustrating an example of the configuration of a cancer treatment apparatus according to a third embodiment;
FIG. 18 is a cross-sectional view illustrating an overview of magnetic field generators of FIG. 17;
FIG. 19 is a cross-sectional oblique view illustrating an overview of the magnetic field generators of FIG. 17;
FIG. 20 is a view illustrating an overview of a cancer treatment apparatus according to a fourth embodiment; and
FIG. 21 is a view illustrating an example in which an airflow is delivered to a patient by an air blower of FIG. 20.

### DESCRIPTION OF THE EMBODIMENTS

For example, when an alternating magnetic field having a frequency of 100 kHz or higher is applied to a living body, heat generation occurs due to an induced current in the living body. When the site of the living body, which is the target of magnetic field application, has a large size, a coil needs to have a large outer size (winding diameter). This causes a high induced current in the living body, and makes the amount of heat generation high. Hence, for example, when treating a diseased site that is present in the head or the abdomen using a coil situated around the head or the abdomen, it is difficult to apply an alternating magnetic field having a frequency, at which a sufficient antitumor effect can be obtained, to the diseased site for a previously prescribed period of time, due to constraints regarding the amount of heat generation.

An object of the present disclosure is to enable applying an alternating magnetic field having a frequency, at which a sufficient antitumor effect can be obtained, to a living body for a previously prescribed period of time while restricting the amount of heat generation from the living body.

According to the present disclosure, it is possible to enable applying an alternating magnetic field having a frequency, at which a sufficient antitumor effect can be obtained, to a living body for a previously prescribed period of time while restricting the amount of heat generation from the living body.

Embodiments for carrying out the embodiments of the present disclosure will be described below in detail with reference to the drawings. The same components in the drawings will be denoted by the same reference numerals, and overlapping descriptions of such components will be omitted where appropriate. For example, the shapes of the components, the size proportions and the relative positional relationships between the components, and the like illustrated in the drawings, to which reference will be made in the following description, are mere examples, and the contents illustrated in the drawings are non-limiting. Moreover, there may be cases where, for example, the components and the like are exaggerated in order to make the description clear.

### <First embodiment>

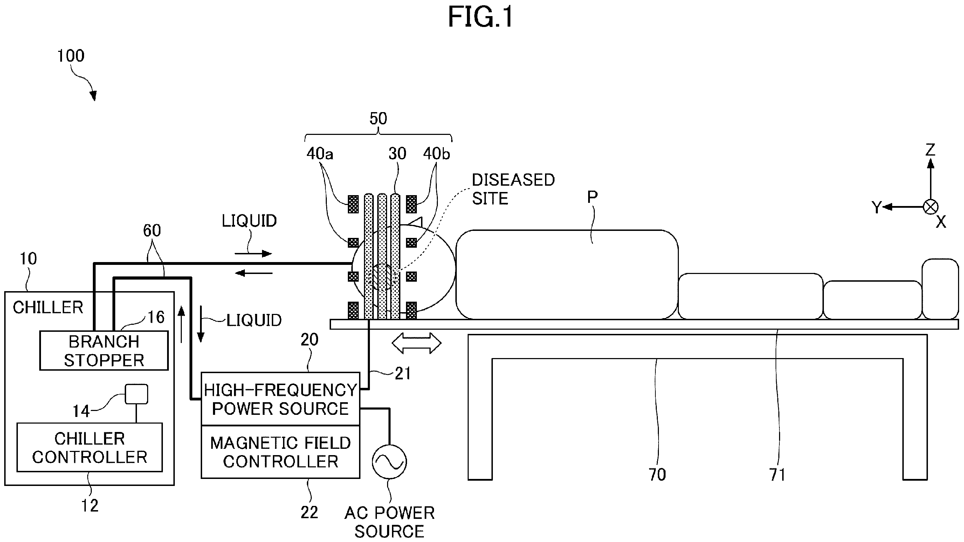

FIG. 1 is a view illustrating an example of the configuration of a cancer treatment apparatus according to a first embodiment. A cancer treatment apparatus 100 illustrated in FIG. 1 is a magnetic therapy apparatus configured to apply an alternating magnetic field to, for example, tumor cells, and expected to inhibit proliferation of cancer cells by this exact application of the alternating magnetic field. The cancer treatment apparatus 100 is different from, for example, a hyperthermia apparatus configured to apply a radio wave or the like and raise the temperature of tumor cells.

The cancer treatment apparatus 100 includes a chiller 10, a high-frequency power source 20, a magnetic field controller 22, and a magnetic field generator 50 including a coil 30, and cores 40a and cores 40b. The chiller 10, the high-frequency power source 20, and the magnetic field controller 22 are situated near a bed 70 on which a patient P lies. The patient P is an example of the living body.

The chiller 10 is a cooling liquid circulating device, and includes a chiller controller 12, a temperature sensor 14, and a branch stopper 16. The chiller 10 is configured to adjust the temperature of a cooling liquid to be supplied to the high-frequency power source 20 and the coil 30 to a previously set suitable temperature based on the temperature detected by the temperature sensor 14.

In addition to the temperature sensor 14, a temperature sensor to be pasted on the patient P, a temperature sensor to be situated near the coil 30, or a thermoviewer (thermography camera) configured to measure the body temperature of the patient P may be provided. The chiller 10 may adjust the temperature of the liquid based not only on the temperature detected by the temperature sensor 14 but on the body temperature of the patient P or the temperature of the coil 30.

Moreover, the chiller 10 may also adjust the flow rate of the liquid to be circulated, while adjusting the temperature of the liquid. In a case where the temperature detected by the temperature sensor 14 becomes higher than a predetermined value, the chiller 10 may output an instruction to the high-frequency power source 20 to instruct the high-frequency power source 20 to reduce a current to apply. The branch stopper 16 is configured to output the liquid that is output from the chiller 10 into respective hoses 60 in a branched state. The hoses 60 are connected to the high-frequency power source 20 and the coil 30, respectively, in order to circulate the liquid between the chiller 10 and the coil 30, and between the chiller 10 and the high-frequency power source 20. The hoses 60 may be, for example, inflexible pipes or the like.

The temperature of the coil 30 is adjusted to, for example, a temperature lower than the temperature (e.g., from 42°C through 43°C) of tumor cells in a treatment using a hyperthermia apparatus, and is desirably adjusted to a temperature lower than the body temperature (e.g., from 35°C through 38°C) of the patient P to whom an alternating magnetic field is applied. It is more desirable to control the temperature of the coil 30 to be approximately room temperature (from 20°C through 30°C) of the room in which the cancer treatment apparatus 100 is situated. In order to inhibit dew condensation, the temperature of the coil 30 is preferably higher than or equal to the dew point in the apparatus environment of the cancer treatment apparatus 100 during application of an alternating magnetic field.

Effects applied to the patient P due to the coil 30 during application of an alternating magnetic field include: rising of the body temperature of the patient P in response to the heat of the coil 30, which has risen above room temperature due to an applied current, being transmitted to the patient P; and heat generation due to a current induced in the body of the patient P in response to the alternating magnetic field from the coil 30. By cooling the coil 30 to a temperature lower than the body temperature of the patient P by means of the circulating liquid, it is possible not only to inhibit the temperature in a region of the patient P to which the alternating magnetic field is applied from rising due to the heat of the coil 30, but to make the cooled coil 30 function as a cooling medium. By inhibiting rising of the temperature in the region of the patient P to which the alternating magnetic field is applied, it is possible to apply a higher current to the coil 30 and increase the intensity of the alternating magnetic field applied to the diseased site. This can improve the antitumor effect and shorten the treatment time.

Either or both of the flow rate and the temperature of the fluid may be adjusted based on the temperature that is sensed by the temperature sensor situated on the body surface of the patient P or on the coil 30, or by a thermoviewer. The treatment using the cancer treatment apparatus 100, in which an alternating magnetic field is applied to the patient P, is at a risk that the temperature of any metallic material embedded in the body such as a stent or the like may rise due to the magnetic field. Hence, it is desirable to equip the cancer treatment apparatus 100 with a metal detector configured to detect any metallic material in the body.

The liquid supplied from the chiller 10 to the high-frequency power source 20 and the coil 30 is not limited to water, and may be oils and various types of aqueous solutions. A non-conductive liquid such as a fluorine-based organic compound and the like may be used in order to minimize trouble in the event of leakage of the liquid. When heat generation from the coil 30 is low, a gas such as air may be supplied to the coil 30.

The high-frequency power source 20 is configured to convert power supplied from an Alternating-Current (AC) power source such as a commercial power source to a high-frequency current of approximately from 100 kHz through 400 kHz in accordance with control of the magnetic field controller 22. The high-frequency current is applied to the coil 30 through a cable 21, to induce an alternating magnetic field from the coil 30. It has been experimentally confirmed that a high antitumor effect is obtained when the high-frequency current applied to the coil 30 is from 200 kHz through 250 kHz.

The magnetic field controller 22 is configured to control the intensity of an alternating magnetic field to be induced from the coil 30 by controlling the frequency, the amplitude, and the application time of the high-frequency current generated by the high-frequency power source 20. The frequency, the amplitude, and the application time of the high-frequency current, which the magnetic field controller 22 controls the high-frequency power source 20 to generate, are previously programmed before application of an alternating magnetic field to the patient P. The frequency, the amplitude, and the application time of the high-frequency current, which the magnetic field controller 22 controls the high-frequency power source 20 to generate, may be controlled such that the intensity of an alternating magnetic field applied to the patient P is fine-tuned in accordance with the degree of rising of the body temperature of the patient P.

Although not illustrated, a matching circuit may be situated between the high-frequency power source 20 and the coil 30, and a current from the high-frequency power source 20 may be applied to the coil 30 via the matching circuit. In this case, it is possible to generate a high current having a high frequency efficiently.

The bed 70 is equipped with a slider 71 configured to enable the patient P to be moved toward the magnetic field generator 50, and is situated near the magnetic field generator 50. The bed 70 is made of a non-magnetic, non-conductive material so as not to be affected by an alternating magnetic field generated by the magnetic field generator 50. By the slider 71 enabling the patient P to be moved toward the magnetic field generator 50, it is possible to adjust the relative positions of the diseased site of the patient P and the coil 30 and apply a suitable alternating magnetic field to the diseased site without stressing the patient P.

In the example illustrated in FIG. 1, the patient P has a diseased site (a tumor due to cancer cells) in the head. Hence, the coil 30 is formed in a size into which the head of the patient P moved together with the slider 71 can be inserted. That is, the inner diameter of the coil 30 is greater than the outer contour of the head of the patient P having the diseased site.

The magnetic field generator 50 may be situated to match the position of a diseased part in anywhere other than the head. For example, when an arm, a leg, or the like has a diseased site, it is possible to insert the diseased site into the space inside the coil 30 and treat the diseased site. The coil 30 may also be formed in a size (inner diameter) matching the shape of the diseased site to which an alternating magnetic field is applied. By forming the coil 30 of the magnetic field generator 50 in various shapes, it is possible to apply an alternating magnetic field having a predetermined intensity to also a diseased site present at a deep position in the body.

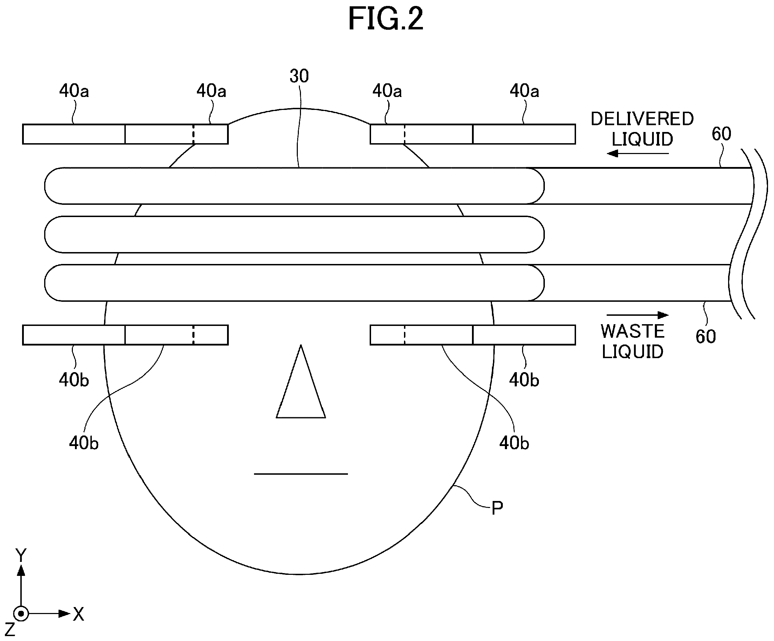

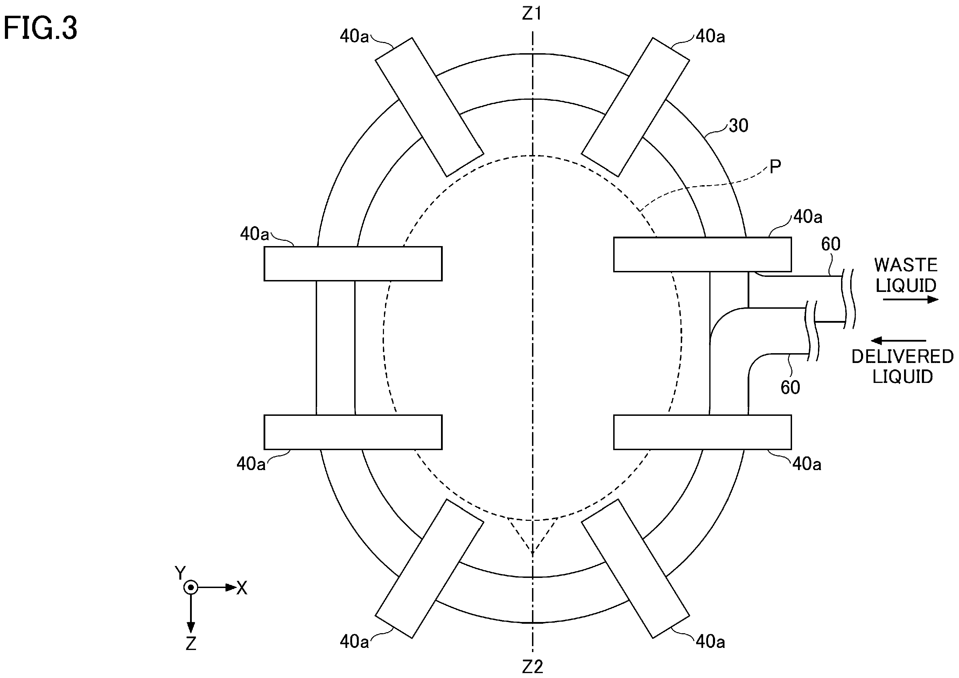

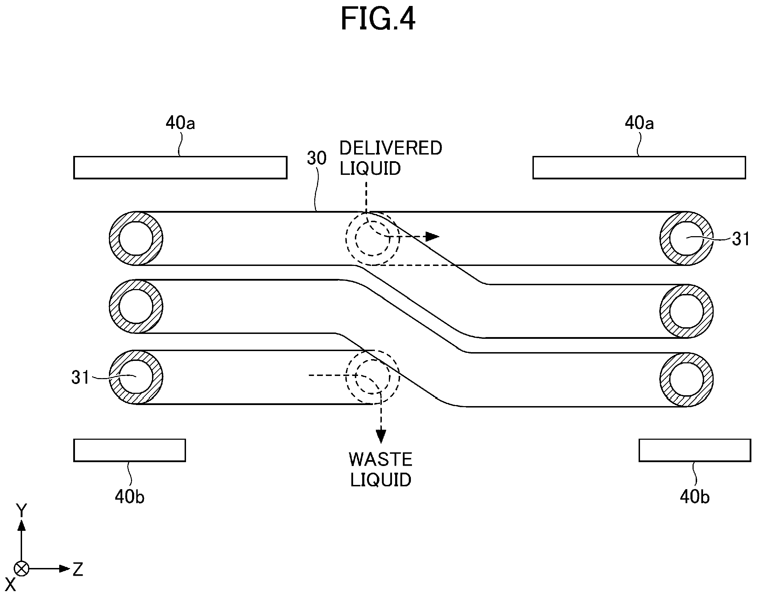

FIG. 2 is a front view illustrating an overview of the magnetic field generator 50 of FIG. 1. FIG. 3 is a top view illustrating an overview of the magnetic field generator 50 of FIG. 1. FIG. 4 is a cross-sectional view of the magnetic field generator 50 along a line Z1-Z2 in FIG. 3. The coil 30 is made of a pipe-shaped conductive material including a hollow portion 31 and having a circular transverse-sectional shape. In the present specification, a transverse section of the coil (or the conductive material of the coil) is defined as a cross-section orthogonal to the longer direction of the conductive material.

The liquid (delivered liquid) supplied from the chiller 10 through the hose 60 is supplied to the hollow portion 31 at one end of the coil 30. The liquid flowing through the hollow portion 31 of the coil 30 absorbs heat generated from the coil 30, and is returned to the chiller 10 through the hose 60 as a waste liquid.

The coil 30 is made of a highly conductive material that has a low loss at a high frequency, such as copper or the like. The number of turns in the coil 30 is 3, but the coil 30 may be formed in a number of turns of approximately from 2 through 5. The cores 40a and the cores 40b are situated on the respective sides of the coil 30 in the coil axial direction, in order to reduce the size of the apparatus while inhibiting any effects on other devices by concentrating the magnetic flux generated from the coil 30 and reducing leakage of the magnetic field to the surroundings of the magnetic field generator 50. By a high-frequency current being flowed through the coil 30, an alternating magnetic field is generated.

If too many cores 40a and cores 40b are situated on the path of the magnetic field, the inductance of the coil 30 becomes excessively high, which may make power source designing and selection difficult. Hence, it is desirable to situate the plurality of cores 40a by spacing them as illustrated in FIG. 3. It is also desirable to situate the plurality of cores 40b by spacing them.

The intensity of an alternating magnetic field applied to the cancer cells in the diseased site of the patient P is 2 mT or higher, and preferably 10 mT or higher. In order to obtain an antitumor effect, it is desirable to apply an alternating magnetic field of 10 mT or higher to the diseased site continuously for 30 minutes or longer every day or every few days. It has come to be experimentally made clear that the cancer cell proliferation inhibiting effect is higher as the intensity of the alternating magnetic field is higher. However, in a case of applying an intense alternating magnetic field to the diseased site from the coil 30 having an inner diameter greater than the outer contour of the body part including the diseased site, there is a risk that the temperature in the region of the patient P to which the alternating magnetic field is applied may exceed a normal range (e.g., 41°C or lower). It does not follow that increasing the intensity of the alternating magnetic field without limit is good, and it is desirable to restrict the intensity of the alternating magnetic field to be lower than 40 mT, and, if possible, 30 mT or lower.

However, as described above, by cooling the coil 30 by means of the circulating liquid, it is possible not only to inhibit the temperature in the region of the patient P to which the alternating magnetic field is applied from rising due to the heat of the coil 30, but to make the cooled coil 30 function as a cooling medium. By inhibiting rising of the temperature in the region of the patient P to which the alternating magnetic field is applied, it is possible to apply a higher current to the coil 30 and increase the intensity of the alternating magnetic field applied to the diseased site. This can improve the antitumor effect and shorten the treatment time.

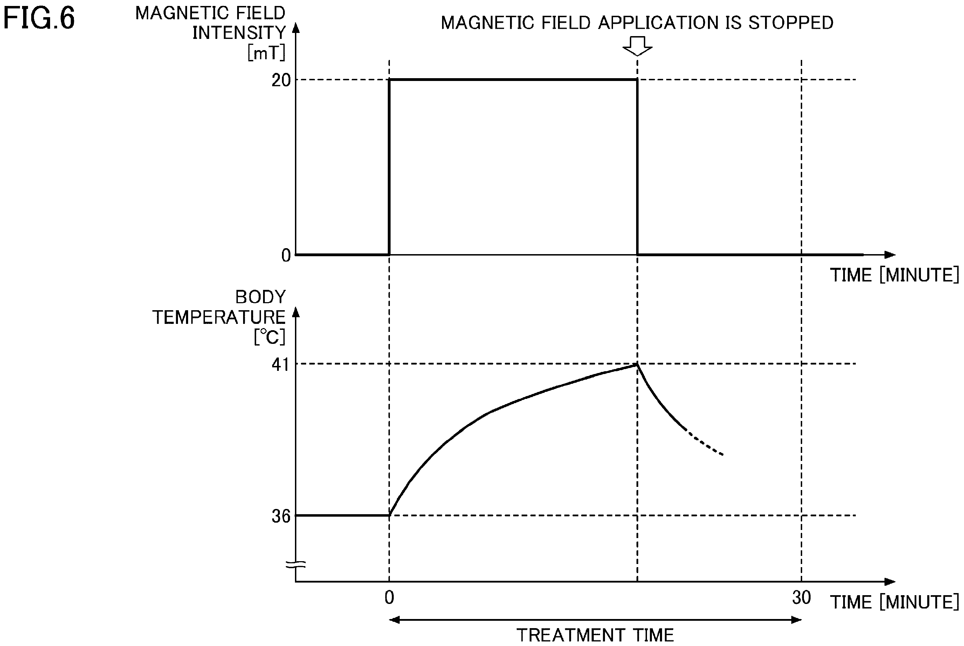

In the present embodiment, the magnetic field controller 22 does not continue applying a maximum magnetic field for as long as the treatment time in which an alternating magnetic field is applied to the patient P, but performs control to adjust the intensity of the alternating magnetic field to be applied to be lower in the initial period of the treatment time than in the ending period of the treatment time. The control performed by the magnetic field controller 22 on the intensity of the alternating magnetic field will be described with reference to FIG. 6 to FIG. 10.

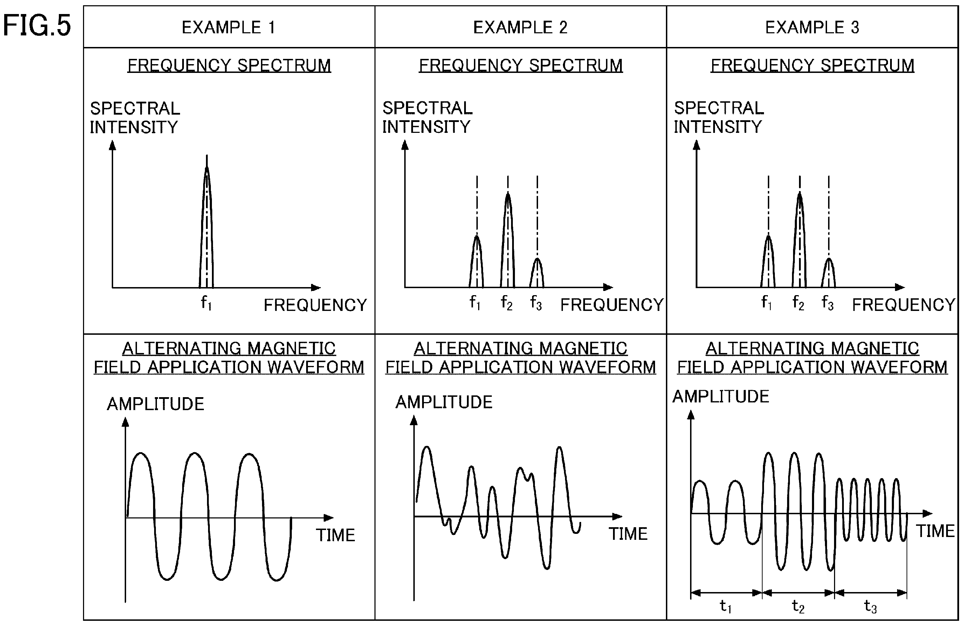

FIG. 5 is a drawing illustrating examples of waveforms of alternating magnetic fields applied to the patient by the cancer treatment apparatus 100 of FIG. 1. It is possible to appropriately change the waveforms of alternating magnetic fields to be applied to the diseased site of the patient in accordance with the types of the cancer cells to be treated.

For example, as illustrated in Example 1, the frequency spectrum of an alternating magnetic field may include a peak of one frequency f1. Alternatively, as illustrated in Example 2 and Example 3, the frequency spectrum of an alternating magnetic field may include peaks of a plurality of frequencies (in these examples, three frequencies f1, f2, and f3).

In Example 1, the alternating magnetic field has a waveform repeating a high level and a low level at a constant cycle. In Example 2, by superimposing the three frequencies f1, f2, and f3, it is possible to provide the alternating magnetic field with a complicated waveform in which unit waveform patterns of the frequencies f1, f2, and f3 are superimposed. In Example 3, by switching among the three frequencies f1, f2, and f3 once in every predetermined period t1, t2, or t3, it is possible to switch among the frequencies of alternating magnetic fields.

As described above, the antitumor effect is higher as the intensity of the alternating magnetic field applied to the patient P is higher. However, because the body temperature would rise due to heat generation due to an induced current, the intensity of the alternating magnetic field has an upper limit until which the alternating magnetic field can be applied to the patient P. It is said that if a human body exceeds 45°C even for a short period of time, the cells undergo an irreversible biochemical reaction and cannot be restored. Hence, it is necessary to set the intensity of the alternating magnetic field to be applied to the diseased site of the patient P, taking into consideration rising of the body temperature due to an induced current. In the following description, for example, the upper limit of the body temperature that would rise in response to application of an alternating magnetic field is set to 41°C.

Regarding the amount of heat generation in a living body in response to application of an alternating magnetic field, although dependent on, for example, the size of the living body, the positional relationship with respect to the coil 30, and the like, a phenomenon in which the body temperature gradually rose by approximately from 3°C through 5°C has been confirmed in an experiment in which an alternating magnetic field of approximately 15 mT was applied to the diseased site for approximately 30 minutes.

FIG. 6 to FIG. 10 are graphs illustrating examples of relationships between the intensity of an alternating magnetic field generated from the coil 30 of FIG. 1 and the body temperature of the patient. The magnetic field controller 22 of FIG. 1 controls the intensity of the alternating magnetic field. Here, it is assumed that the treatment time, which is an application time for which application of the alternating magnetic field is continued, is 30 minutes. FIG. 6 to FIG. 10 illustrate examples of a method of controlling the cancer treatment apparatus 100 and a cancer treatment method using the cancer treatment apparatus 100.

It has been found that applying an alternating magnetic field to a patient P raises the body temperature due to the effect of the alternating magnetic field. In the meantime, as the body temperature rises in response to application of the alternating magnetic field, the living body, attempting to reduce the body temperature, causes a reaction to promote heat dissipation by, for example, increasing the amount of blood circulation, increasing the amount of perspiration, and the like. Here, the amount of heat dissipation increases not instantly, but gradually over a period of time of approximately a few minutes through a few tens of minutes.

In a case of applying a maximum alternating magnetic field from the very beginning of alternating magnetic field application, at which the amount of heat dissipation is low, there is a risk that heat dissipation cannot catch up and the body temperature may sharply rise. Hence, a relatively weak alternating magnetic field is applied at the beginning of alternating magnetic field application, and the intensity of the alternating magnetic field applied is gradually increased along with the progression of the heat dissipation promoting reaction in the living body. This makes it possible to apply a more intense alternating magnetic field while inhibiting rising of the body temperature. As a result, a better treatment effect can be obtained.

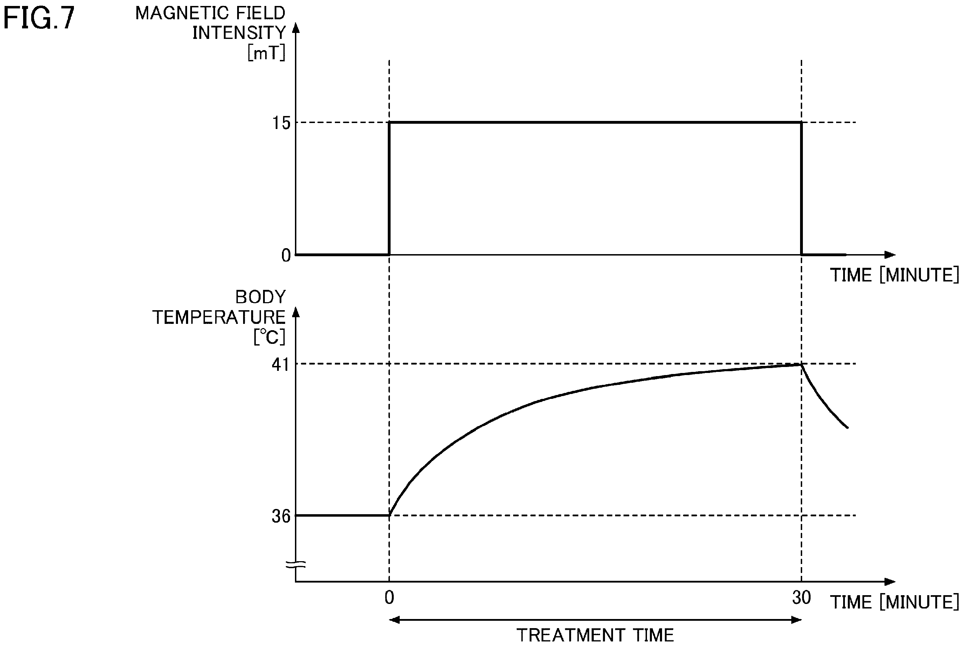

In FIG. 6, an alternating magnetic field of 20 mT was applied to the patient P from the beginning of the treatment time. In this case, the body temperature reached 41°C in the middle of the treatment time. Hence, application of the alternating magnetic field was stopped without waiting for the end timing to end application of the alternating magnetic field to come. The timing to end application of the alternating magnetic field is, for example, 30 minutes after the start of application of the alternating magnetic field. In FIG. 7, an alternating magnetic field of 15 mT was applied to the patient P from the beginning of the treatment time. In this case, the body temperature reached 41°C at the end of the treatment time of 30 minutes, and it was possible to fulfill the thirty minutes, which is the prescribed application time, without exceeding the upper limit of the body temperature of the patient P.

It can be seen that the body temperature rising rate was highest immediately after the start of application of the alternating magnetic field, and then gradually became lower along with application of the alternating magnetic field. This is considered due to the body temperature adjusting reaction of the living body, and, specifically, due to increase in the amount of heat dissipation from the living body due to, for example, vascular relaxation-induced increase in the flow of heat or the like in response to rising of the body temperature due to the alternating magnetic field. The amount of heat dissipation from the living body is considered to gradually increase over time after the start of application of the alternating magnetic field. Depending on the amount of heat dissipation from the living body, the body temperature may be maintained at a constant temperature or may decrease.

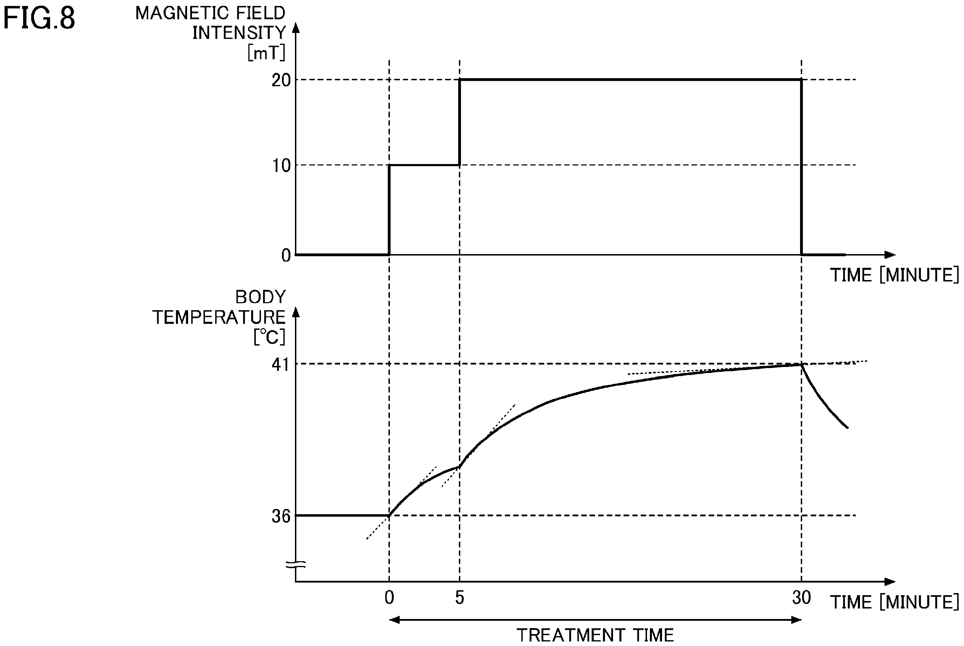

In FIG. 8, an alternating magnetic field of 10 mT was applied to the patient P for 5 minutes from the start of the treatment, and an alternating magnetic field of 20 mT was subsequently applied to the patient P until the end of the treatment. That is, in FIG. 8, the intensity of the alternating magnetic field was increased stepwise. The broken-line tangential lines on the curve indicating the changes in the body temperature indicate the body temperature rising rates (rising gradients). It can be seen that the body temperature rising rate became lower as the body temperature rose. The five minutes from the start of the treatment is an example of a first period of time. The alternating magnetic field of 20 mT is an example of a previously set maximum value.

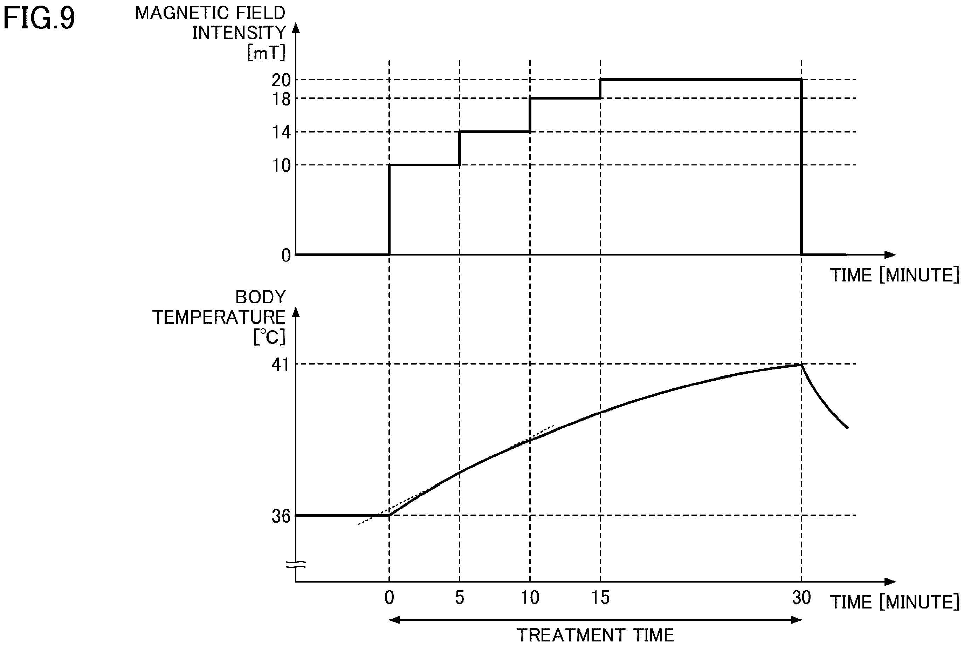

In FIG. 9, an alternating magnetic field of 10 mT was applied to the patient P for 5 minutes from the start of the treatment, and alternating magnetic fields of 14 mT and 18 mT were subsequently applied to the patient P each for 5 minutes. Since the elapse of these 15 minutes, an alternating magnetic field of 20 mT was applied to the patient P. That is, in FIG. 9, the intensity of the alternating magnetic field applied to the patient P was sequentially increased multi-stepwise. It can be seen that the body temperature rising rate in the beginning of the treatment in FIG. 9 was lower than the body temperature rising rate in the beginning of the treatment in FIG. 8. The fifteen minutes from the start of the treatment is an example of the first period of time.

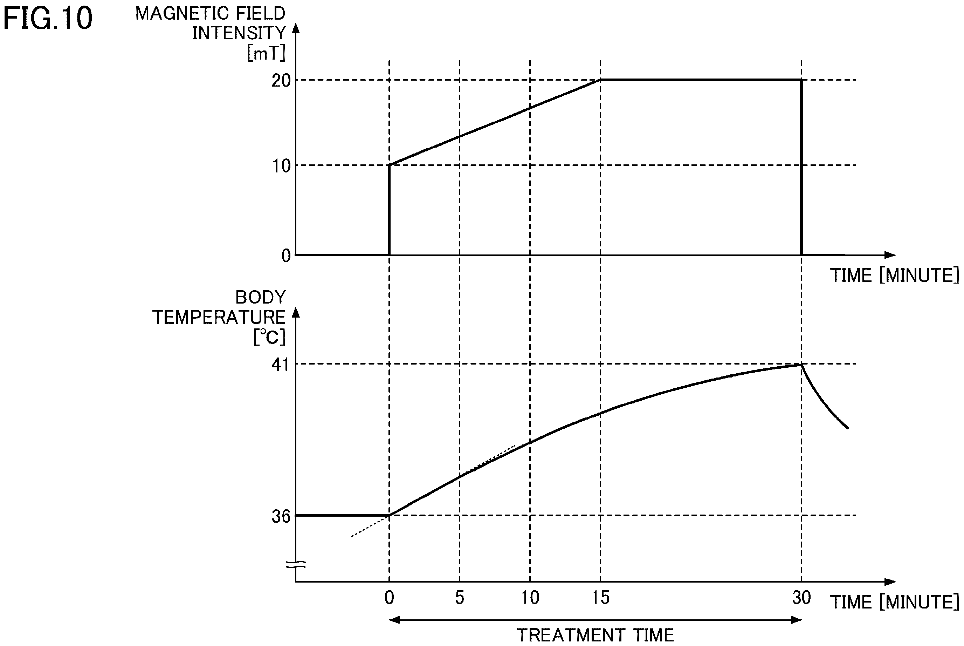

In FIG. 10, an alternating magnetic field was applied to the patient P while continuously changing the intensity of the alternating magnetic field from 10 mT to 20 mT until 15 minutes elapsed from the start of the treatment, and an alternating magnetic field of 20 mT was applied to the patient P since the elapse of the fifteen minutes. Also in FIG. 10, it can be seen that the body temperature rising rate in the beginning of the treatment was lower than the body temperature rising rate in the beginning of the treatment in FIG. 8. The fifteen minutes from the start of the treatment is an example of the first period of time.

As illustrated in FIG. 8 to FIG. 10, by adjusting the intensity of the alternating magnetic field to be applied to be lower than 20 mT, which is the maximum intensity, for as long as a period in which the amount of heat dissipation from the living body is low because of the period being immediately after the start of application of the alternating magnetic field, it is possible to wait for the amount of heat dissipation to increase while inhibiting rising of the body temperature. Then, by increasing the intensity of the alternating magnetic field synchronously with increase in the amount of heat dissipation, it is possible to apply an alternating magnetic field of 20 mT to the patient P from middle of the treatment time while controlling the body temperature to be lower than or equal to 41°C, which is the upper limit.

It was confirmed possible to control the body temperature to 41 °C or lower by sufficiently increasing the amount of heat dissipation during the treatment time, as long as the intensity of the alternating magnetic field immediately after the start of the treatment was, for example, approximately lower than or equal to 80% (16 mT), preferably lower than or equal to 60% (12 mT) of the intensity of the alternating magnetic field in the last half of the treatment time. This makes it possible to apply a higher alternating magnetic field to the patient P ultimately and to increase the antitumor effect better than in a case of continuing to apply an alternating magnetic field of 15 mT to the patient P from the beginning of the treatment time.

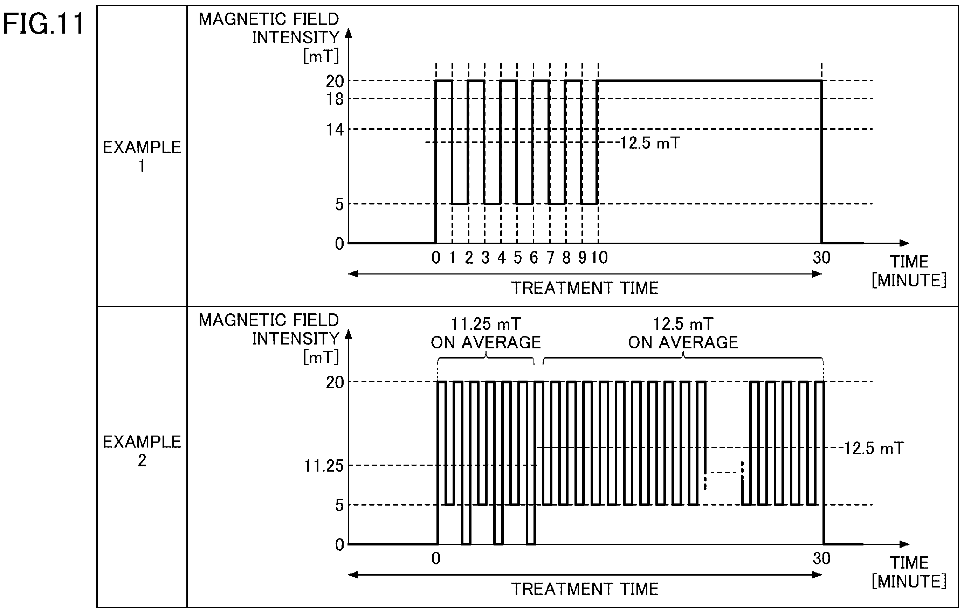

FIG. 11 is a graph illustrating another example in which the intensity of the alternating magnetic field generated from the coil 30 of FIG. 1 is changed during the treatment time. Also in FIG. 11, the treatment time, which is the alternating magnetic field application time, is assumed to be 30 minutes. In FIG. 11, the magnetic field controller 22 increases the average of the intensity of the alternating magnetic field over time during the treatment time from the start until the end timing of application of the alternating magnetic field to the patient P.

In Example 1 illustrated in FIG. 11, alternating magnetic fields of 20 mT and 5 mT were alternately applied to the patient P for 10 minutes from the start of the treatment, and an alternating magnetic field of 20 mT was subsequently applied to the patient P until the end of the treatment. In this case, an alternating magnetic field of 12.5 mT on average was applied for 10 minutes from the start of the treatment, and an alternating magnetic field of 20 mT on average was subsequently applied.

In Example 2 illustrated in FIG. 11, alternating magnetic fields of 20 mT, 5 mT, 20 mT, and 0 mT were repeatedly applied to the patient P for a predetermined period of time from the start of the treatment, and alternating magnetic fields of 20 mT and 5 mT were alternately applied to the patient P subsequently until the end of the treatment. The alternating magnetic field of 0 mT means to stop application. In this case, an alternating magnetic field of 11.25 mT on average was applied for the predetermined period of time from the start of the treatment, and an alternating magnetic field of 12.5 mT on average was subsequently applied. An alternating magnetic field of 20 mT on average may optionally be applied in the last half of the treatment time.

By switching alternating magnetic fields to be applied to the patient P during the treatment time such that the average per unit time increases as illustrated in FIG. 11, it is possible to wait for the amount of heat dissipation to increase while inhibiting rising of the body temperature during the treatment time just the same as in FIG. 8 to FIG. 10. Then, by increasing the intensity of the alternating magnetic field to be applied, synchronously with increase in the amount of heat dissipation, it is possible to apply an alternating magnetic field having a sufficient intensity to the patient P while controlling the body temperature to be lower than or equal to 41 °C.

In the first embodiment, it is possible to wait for the amount of heat dissipation to increase while inhibiting rising of the body temperature, by adjusting the intensity of the alternating magnetic field to be applied to be lower than 20 mT, which is the maximum intensity, for as long as a period in which the amount of heat dissipation from the living body is low because of the period being after the start of application of the alternating magnetic field. Then, by increasing the intensity of the alternating magnetic field to be applied, synchronously with increase in the amount of heat dissipation, it is possible to apply an alternating magnetic field of 20 mT to the patient P from the middle of the treatment time while controlling the body temperature to be lower than or equal to 41°C, which is the upper limit. As a result, it is possible to apply an alternating magnetic field having a frequency, at which a sufficient antitumor effect can be obtained, to the patient P for a previously prescribed treatment time, while restricting the amount of heat generation from the living body.

### <Second embodiment>

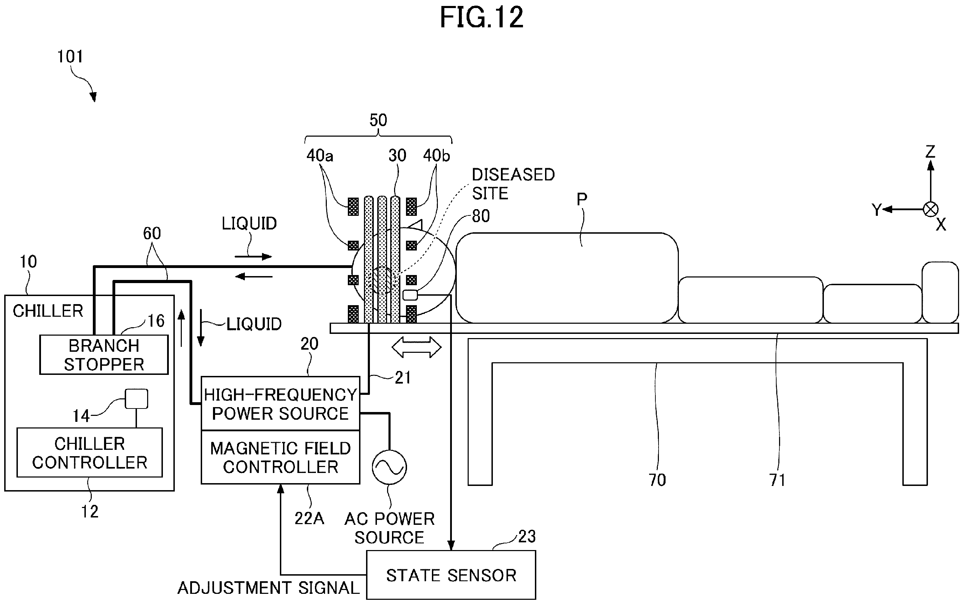

FIG. 12 is a view illustrating an example of the configuration of a cancer treatment apparatus according to a second embodiment. Any configurational particulars and functions that are the same as or similar to those in the first embodiment will be denoted by the same reference numerals, and detailed descriptions of such will be omitted. An alternating magnetic field generated by a cancer treatment apparatus 101 illustrated in FIG. 12 is the same as the alternating magnetic field generated by the cancer treatment apparatus 100 illustrated in FIG. 1. That is, the cancer treatment apparatus 101 is configured to apply any of the alternating magnetic fields illustrated in FIG. 8 to FIG. 11 to the patient P in the treatment time.

The cancer treatment apparatus 101 illustrated in FIG. 12 includes a magnetic field controller 22A instead of the magnetic field controller 22 of the cancer treatment apparatus 100 illustrated in FIG. 1, and includes a state sensor 23 configured to sense a heat generating state of the patient P in addition to the components of the cancer treatment apparatus 100 illustrated in FIG. 1. The state sensor 23 may be provided in the magnetic field controller 22A.

The state sensor 23 is configured to sense the heat generating state of the patient P by monitoring one or a plurality selected from body temperature, breathing, pulse, heart beats, and blood flow rate of the patient P. In FIG. 12, the state sensor 23 senses the body temperature of the patient P as the heat generating state from a temperature sensor 80 pasted near a region of the patient P to which an alternating magnetic field is applied. For example, the temperature sensor 80 is an optical probe sensor that is unsusceptible to magnetic fields. Instead of the temperature sensor 80 that is directly pasted on the patient P, the cancer treatment apparatus 101 may be equipped with an infrared thermometer, a thermoviewer, or the like that is configured to sense the body temperature of the patient P contactlessly. In this case, the state sensor 23 senses the body temperature sensed by the thermoviewer or the like as the heat generating state.

The state sensor 23 senses or estimates the body temperature of the patient P based on the heat generating state of the patient P sensed. The state sensor 23 determines whether the intensity of the alternating magnetic field is suitable or not with respect to the amount of heat dissipation, based on the body temperature sensed or estimated and a temporal body temperature changing rate, and outputs an adjustment signal instructing adjustment of the intensity of the alternating magnetic field to the magnetic field controller 22A.

Specifically, the state sensor 23 outputs an adjustment signal instructing reducing the intensity of the alternating magnetic field to the magnetic field controller 22A in a case where the probability of the body temperature reaching 41°C, which is the threshold, is high because it can be estimated that the body temperature rising rate is high and that the amount of heat dissipation is low. The state sensor 23 outputs an adjustment signal instructing increasing the intensity of the alternating magnetic field to the magnetic field controller 22A in a case where the probability of the body temperature reaching 41 °C, which is the threshold, is low because the body temperature rising rate is low and the amount of heat dissipation is high.

It is assumed that a threshold, which is of the breathing rate, the pulse rate, the number of heart beats, or the blood flow rate, in a case of the body temperature reaching 41°C is previously known. In this case, the state sensor 23 may output an adjustment signal instructing reducing the intensity of the alternating magnetic field to the magnetic field controller 22A in a case where the probability of the breathing rate, the pulse rate, the number of heart beats, or the blood flow rate exceeding the threshold is high.

The magnetic field controller 22A controls the high-frequency power source 20 in accordance with previously programmed specifications regarding outputting of the high-frequency current, such as the frequency, the amplitude, the application time, and the like (i.e., specifications regarding the temporal change in the intensity of the alternating magnetic field), just the same as the magnetic field controller 22 illustrated in FIG. 1. Moreover, the magnetic field controller 22A has a function for adjusting the intensity of the alternating magnetic field applied to the patient P based on the adjustment signal from the state sensor 23. Hence, the magnetic field controller 22A can fine-tune the curves illustrated in FIG. 8 to FIG. 11 indicating the changes in the intensity of the alternating magnetic field in accordance with actual changes in the body temperature of the patient P. As a result, for example, it is possible to apply an alternating magnetic field having a suitable intensity to the diseased site in accordance with the physical conditions of the patient P on the day concerned.

Also in the second embodiment, it is possible to apply an alternating magnetic field having a frequency, at which a sufficient antitumor effect can be obtained, to the patient P for a previously prescribed period of time while restricting the amount of heat generation from the living body as in the first embodiment.

Moreover, in the second embodiment, it is possible to fine-tune the curves indicating the changes in the intensity of the alternating magnetic field, which are previously programmed, in accordance with actual changes in the body temperature of the patient P. As a result, it is possible to apply an alternating magnetic field having a suitable intensity to the diseased site in accordance with the physical conditions of the patient P on the day concerned.

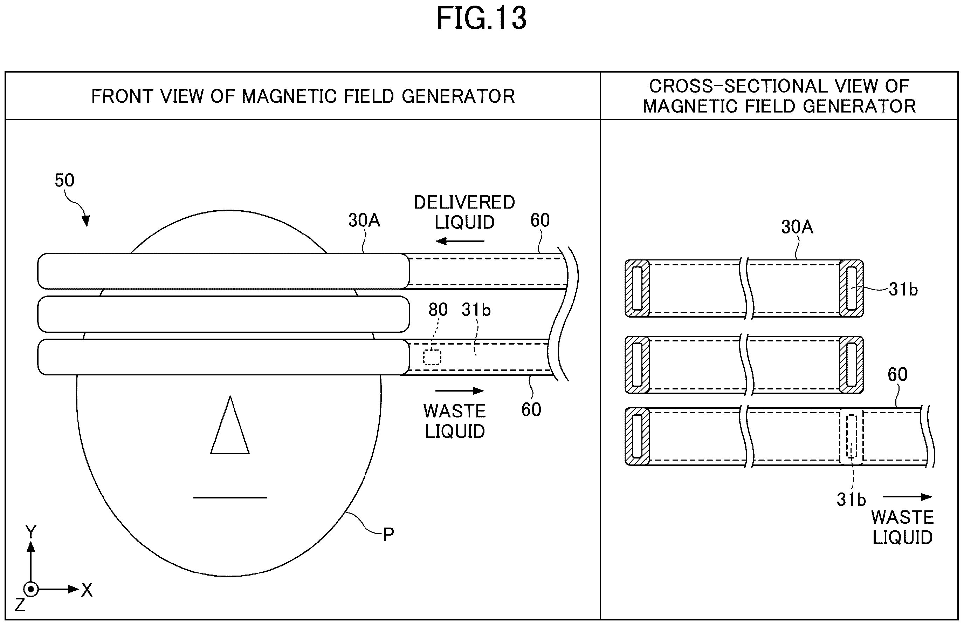

FIG. 13 is a view illustrating another example of the magnetic field generator 50 mounted on the cancer treatment apparatuses 100 and 101 of FIG. 1 and FIG. 12. The magnetic field generator 50 illustrated in FIG. 13 has the same configuration and functions as those of the magnetic field generator 50 illustrated in FIG. 1, except that it includes a coil 30A instead of the coil 30. In FIG. 13, the cores 40a and the cores 40b illustrated in FIG. 2 are omitted. However, for example, the cores 40a and the cores 40b may be situated on the respective sides of the coil 30A in the coil axial direction.

The coil 30A is made of a conductive material such as copper or the like, which has a rectangular transverse-sectional shape having shorter sides and longer sides and includes a hollow portion 31b. The coil 30A has what is generally referred to as a flatwise-wound shape obtained by winding the conductive material in a state such that a surface of the conductive material having a dimension that formed by a longer side of the rectangular transverse-sectional shape faces inward. Hence, it is possible to make a facing area, over which the coil 30A faces the body part of the patient P to which an alternating magnetic field is applied, larger than a facing area, over which a coil made of a material having a circular transverse-sectional shape faces that body part, and to improve the efficiency with which the body part to which the alternating magnetic field is applied is cooled. As a result, it is possible to inhibit rising of the body temperature of the body part to which the alternating magnetic field is applied, and to perform a more effective treatment.

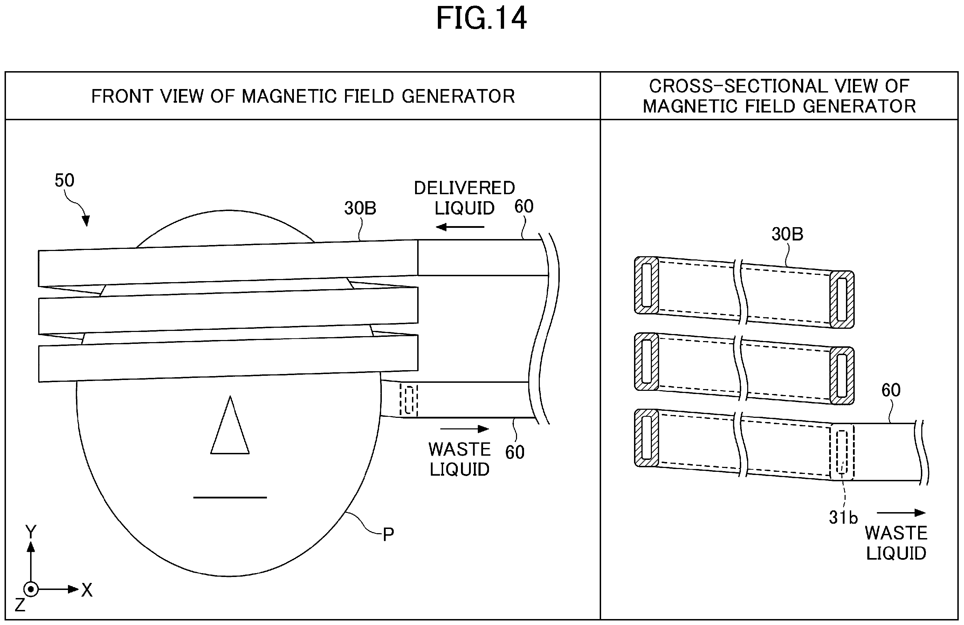

FIG. 14 is a view illustrating still another example of the magnetic field generator 50 mounted on the cancer treatment apparatuses 100 and 101 of FIG. 1 and FIG. 12. The magnetic field generator 50 illustrated in FIG. 14 has the same configuration and functions as those of the magnetic field generator 50 illustrated in FIG. 13, except that it includes a coil 30B instead of the coil 30A. In FIG. 14, the cores 40a and the cores 40b illustrated in FIG. 2 are omitted. However, for example, the cores 40a and the cores 40b may be situated on the respective sides of the coil 30B in the coil axial direction.

The coil 30B has the same shape as that of the coil 30A of FIG. 13, except that it is wound helically. That is, the coil 30B is made of a conductive material including a hollow portion 31b having a rectangular cross-sectional shape having shorter sides and longer sides. The coil 30B is wound in a state such that a surface of the conductive material having a dimension that is formed by a longer side of the rectangular cross-sectional shape faces inward. By winding the coil 30B helically, it is possible to avoid the coil being bent at some portions as illustrated in, for example, FIG. 4. Hence, it is possible to form an inexpensive coil 30B easily.

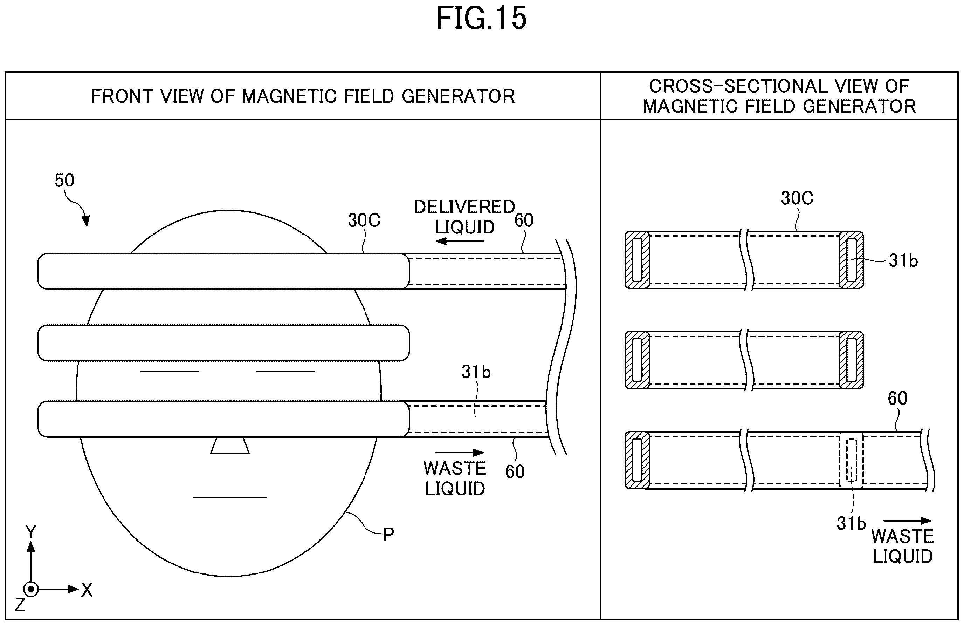

FIG. 15 is a view illustrating another example of the magnetic field generator 50 mounted on the cancer treatment apparatuses 100 and 101 of FIG. 1 and FIG. 12. The magnetic field generator 50 illustrated in FIG. 15 has the same configuration and functions as those of the magnetic field generator 50 illustrated in FIG. 13, except that it includes a coil 30C instead of the coil 30A. In FIG. 15, the cores 40a and the cores 40b illustrated in FIG. 2 are omitted. However, for example, the cores 40a and the cores 40b may be situated on the respective sides of the coil 30C in the coil axial direction.

The interval between conductive portions in the coil 30C per winding is set to be greater than the interval between conductive portions in the coil 30A of FIG. 13 per winding. The other structural particulars of the magnetic field generator 50 are the same as those in the structure of the magnetic field generator 50 of FIG. 13. By making the interval between conductive portions in the coil 30C greater, it is possible to reduce an alternating-current resistance due to a near-field interaction when applying a high-frequency current to the coil 30C. Moreover, for example, it is possible to align the gap between conductive portions to come at the position of the eyes of the patient P. Hence, it is possible to not block the patient P's field of vision, and to reduce uneasiness that may be felt by the patient P during the treatment.

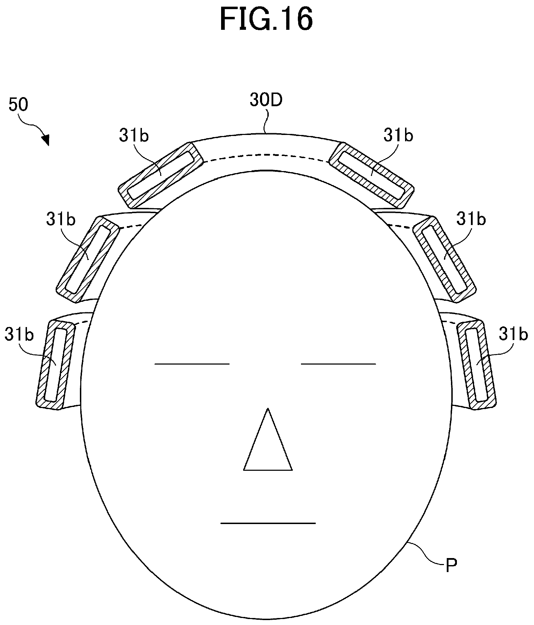

FIG. 16 is a view illustrating another example of the magnetic field generator 50 mounted on the cancer treatment apparatuses 100 and 101 of FIG. 1 and FIG. 12. The magnetic field generator 50 illustrated in FIG. 16 has the same configuration and functions as those of the magnetic field generator 50 illustrated in FIG. 13, except that it includes a coil 30D instead of the coil 30A. In FIG. 16, the cores 40a and the cores 40b illustrated in FIG. 2 are omitted. However, for example, the cores 40a and the cores 40b may be situated along the external surface of the coil 30D.

The coil 30D has an internal surface having a hemispherical shape conforming to the shape of a head. Just the same as the coil 30A of FIG. 13, the coil 30D is made of a conductive material including a hollow portion 31b having a rectangular cross-sectional shape having shorter sides and longer sides, and the coil 30D is wound in a state such that a surface of the conductive material having a dimension that is formed by a longer side of the rectangular cross-sectional shape faces inward.

By shaping the internal surface of the coil 30D, which includes the rectangular hollow portion 31b, in the hemispherical shape conforming to the shape of a head, it is possible to make the distance between: the surface of the coil 30D having the dimension of the longer sides; and the skin of the head short irrespective of the positions of the conductive portions of the coil 30D. As a result, it is possible to further improve the efficiency with which the head to which an alternating magnetic field is applied is cooled, compared with the case of the coil 30A illustrated in FIG. 13. Note that by shaping the internal surface of the coil 30D in the hemispherical shape, it is possible to increase a breast cancer treatment effect.

### <Third embodiment>

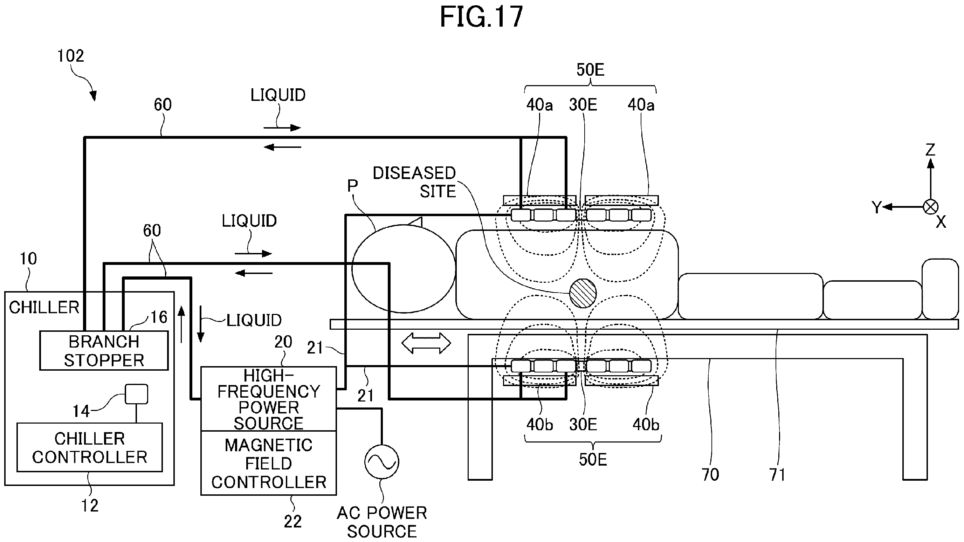

FIG. 17 is a view illustrating an example of the configuration of a cancer treatment apparatus according to a third embodiment. Any configurational particulars and functions that are the same as or similar to those in the first embodiment will be denoted by the same reference numerals, and detailed descriptions of such will be omitted.

The functions of the cancer treatment apparatus 102 illustrated in FIG. 17 are the same as the functions of the cancer treatment apparatus illustrated in FIG. 1. For example, the alternating magnetic field generated by the cancer treatment apparatus 102 is the same as the alternating magnetic field generated by the cancer treatment apparatus 100 illustrated in FIG. 1. That is, the cancer treatment apparatus 102 is configured to apply any of the alternating magnetic fields illustrated in FG. 8 to FIG. 11 to the patient P during the treatment time.

The cancer treatment apparatus 102 illustrated in FIG. 17 includes a pair of magnetic field generators 50E facing each other via the patient P, and, for example, can apply an alternating magnetic field to a diseased site present in the abdomen of the patient P. Each magnetic field generator 50E includes a disk-shaped coil 30E, and cores 40a and cores 40b situated to face the coil 30E. By making the disk-shaped coils 30E face each other via the diseased site, it is possible to apply an intense alternating magnetic field to the diseased site also in a case where the diseased site is located at a deep position in the body.

The chiller 10 has the same configuration and function as those of the chiller 10 of FIG. 1, except that it is configured to circulate a liquid to the high-frequency power source 20 and to both of the pair of coils 30E via the branch stopper 16 in order to supply a liquid having a suitable temperature to the high-frequency power source 20 and to the pair of coils 30E. The high-frequency power source 20 is connected to both of the pair of coils 30E through cables 21, and is configured to output an alternating current to both of the pair of coils 30E in accordance with control of the magnetic field controller 22. The pair of coils 30E are configured to generate alternating magnetic fields in accordance with an alternating current. The functions of the bed 70 and the slider 71 are the same as those of the bed 70 and the slider 71 of FIG. 1. In the present embodiment, the patient P lying on the bed 70 is moved together with the slider 71 to the position at which the abdomen is sandwiched between the pair of magnetic field generators 50E.

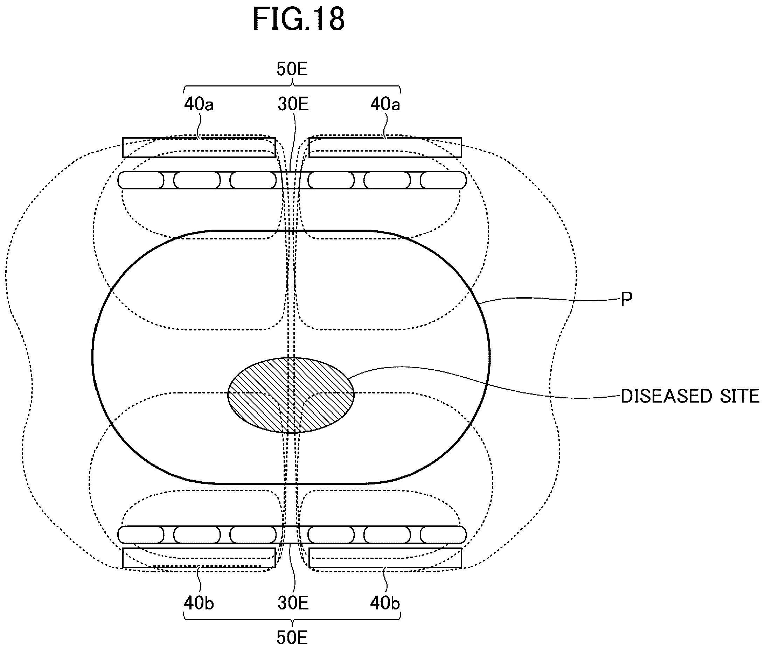

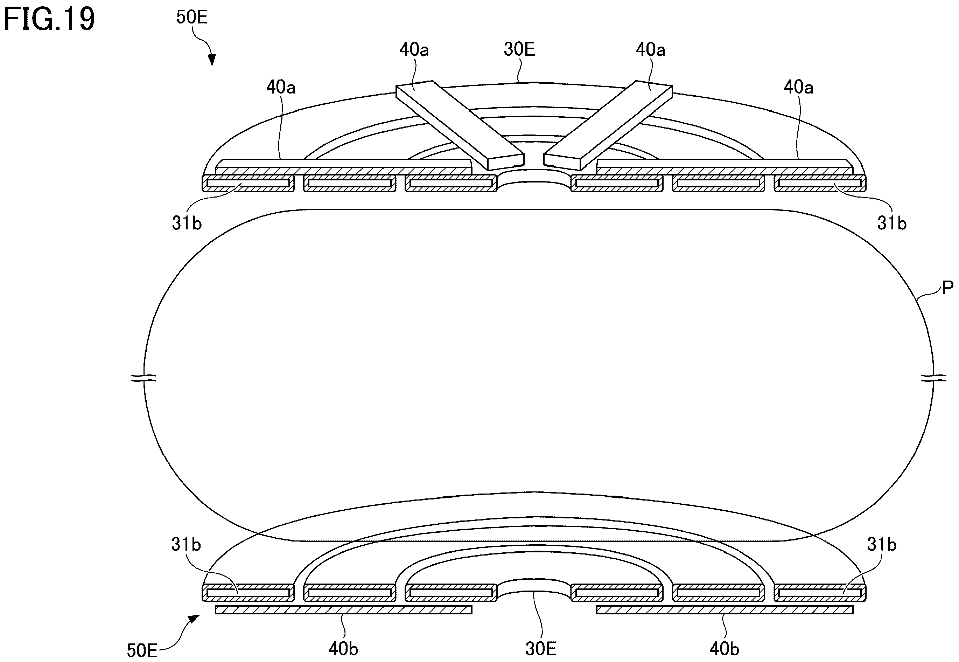

FIG. 18 is a cross-sectional view illustrating an overview of the magnetic field generators 50E of FIG. 17. FIG. 19 is a cross-sectional oblique view illustrating an overview of the magnetic field generators 50E of FIG. 17. The broken-line curves illustrated in FIG. 18 indicate the distribution of the intensity of the alternating magnetic fields generated from the coils 30E. Each coil 30E has what is generally referred to as a spiral shape, and is made of a conductive material that has a rectangular transverse-sectional shape having shorter sides and longer sides and includes a rectangular hollow portion 31b. The pair of coils 30E face each other by their surfaces that have a dimension that is the length of the longer sides of the rectangular transverse-sectional shape. Although non-illustrated, for example, liquid inlet portions are situated at portions of the circumferences of the spiral-shaped coils 30E, and liquid outlet portions are situated at the centers of the spiral-shaped coils 30E.

Hence, it is possible to make a facing area, over which the pair of coils 30E each face the body part of the patient P to which the alternating magnetic fields are applied, larger than a facing area, over which a coil made of a material having a circular transverse-sectional shape faces the body part, and to improve the efficiency with which the body part to which the alternating magnetic fields are applied is cooled. As a result, it is possible to inhibit rising of the body temperature of the body part to which the alternating magnetic fields are applied.

The cores 40a and the cores 40b each have a shape of a flat rectangular-parallelepiped, and are situated over surfaces of the pair of coils 30E opposite to the coils' facing surfaces and having the dimension that is the length of the longer sides of the coils' rectangular transverse-sectional shape, in a state in which the cores 40a are spaced apart in the circumferential direction of the disk-shaped coil 30E and the cores 40b are spaced apart in the circumferential direction of the disk-shaped coil 30E. By situating the cores 40a and the cores 40b in proximity to the coils 30E, it is possible to intensify the alternating magnetic fields to be applied to the diseased site by inhibiting extra leakage of the alternating magnetic fields to the surroundings as illustrated in FIG. 18. Although illustration is omitted, it is possible to situate side cores configured to magnetically connect the cores 40a and the cores 40b that are situated on the side surfaces of the body, such that a closed magnetic circuit is formed to further reduce leaking magnetic fields and apply even more intense alternating magnetic fields to the living body.

Hence, also in the third embodiment, it is possible to apply an alternating magnetic field having a frequency, at which a sufficient antitumor effect can be obtained, to the patient P for a previously prescribed period of time, while restricting the amount of heat generation from the living body as in the first embodiment described above.

### <Fourth embodiment>

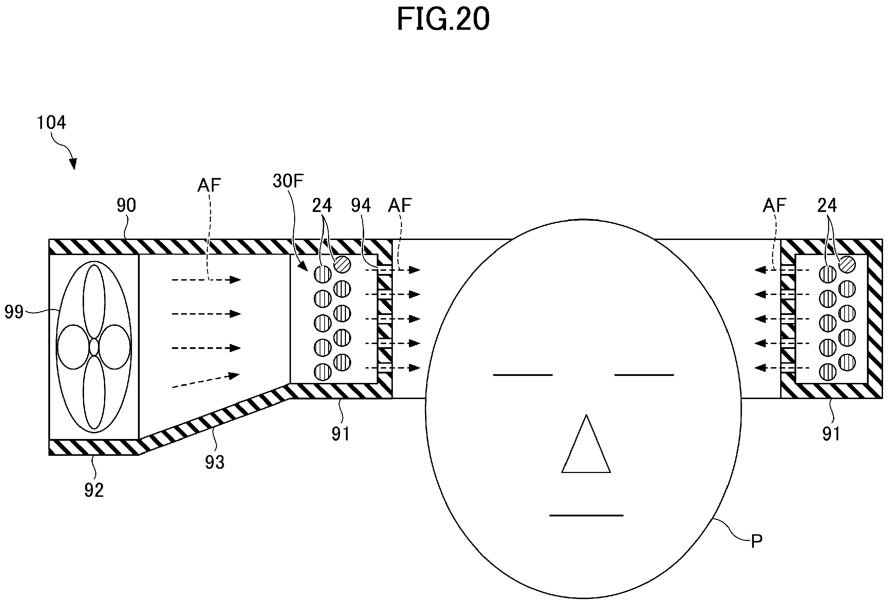

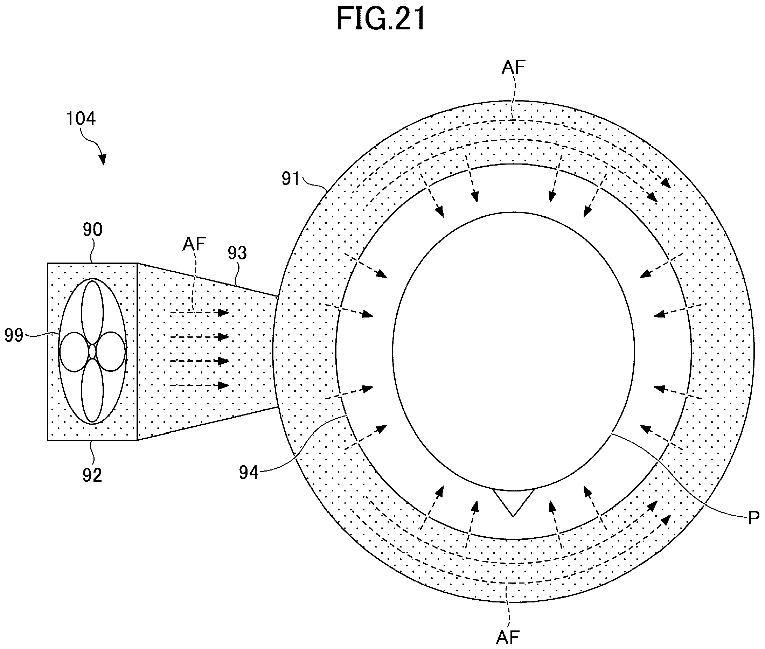

FIG. 20 and FIG. 21 are views illustrating overviews of a cancer treatment apparatus according to a fourth embodiment. The functions of the cancer treatment apparatus 104 illustrated in FIG. 20 are the same as the functions of the cancer treatment apparatus 100 illustrated in FIG. 1. For example, the alternating magnetic field generated by the cancer treatment apparatus 104 is the same as the alternating magnetic field generated by the cancer treatment apparatus 100 illustrated in FIG. 1. That is, the cancer treatment apparatus 104 is configured to apply any of the alternating magnetic fields illustrated in FIG. 8 to FIG. 11 to the patient P during the treatment time.

The cancer treatment apparatus 104 includes a coil 30F formed by winding a non-hollow conductive material 24 such as a litz wire or the like, and an air blower 90 in which the coil 30F is stored. The air blower 90 includes a storage 91 for the coil 30F, an air blowing part 92 in which a fan 99 is situated, and an air passage 93 joining the storage 91 and the air blowing part 92 with each other.

The storage 91 has an annular transverse-sectional shape conforming to the shape of the coil 30F, and includes a plurality of air passage holes 94 in a cylinder-shaped inner wall of the storage 91. An airflow AF of air delivered into the storage 91 from the fan 99 through the air passage 93 cools the conductive material 24 by passing through gaps in the conductive material 24, and is evacuated through the air passage holes 94. The amount of heat generation from the coil 30F made of a litz wire is lower than the amount of heat generation from the hollow coil 30 illustrated in FIG. 1. Hence, should the airflow AF absorb the heat of the coil 30F, no hot air is to be emitted through the air passage holes 94.

For example, the head of the patient P in which a diseased site is present is positioned inside the cylinder-shaped inner wall of the storage 91, and receives an alternating magnetic field generated from the coil 30F. The airflow AF evacuated through the air passage holes 94 touches the head and inhibits rising of the temperature of the head. Air cooled by a heat pump or the like may be delivered to the storage 91 from the air blowing part 92. By having the coil 30F stored in the storage 91 and delivering air to the patient P through the storage 91, the cancer treatment apparatus 104 illustrated in FIG. 20 can cool the coil 30F utilizing the airflow AF for inhibiting rising of the temperature of the patient P.

Hence, also in the fourth embodiment, it is possible to apply an alternating magnetic field having a frequency, at which a sufficient antitumor effect can be obtained, to the patient P for a previously prescribed period of time, while restricting the amount of heat generation from the living body as in the first embodiment.

The present disclosure has been described based on the embodiments. However, the present disclosure is not limited to the requirements specified in the embodiments described above. These requirements can be modified within the range in which the purpose of the present disclosure is not missed, and can be suitably defined in accordance with the form of application.

## Claims

1. A cancer treatment apparatus, comprising:
a magnetic field generator configured to generate an alternating magnetic field to be applied to a living body; and
a magnetic field controller configured to increase an intensity of the alternating magnetic field generated by the magnetic field generator stepwise until a first period of time elapses since beginning application of the alternating magnetic field to the living body, and to set the intensity of the alternating magnetic field to a previously set maximum value from elapse of the first period of time until a timing to end the application of the alternating magnetic field.

2. The cancer treatment apparatus according to claim 1, wherein the magnetic field controller is configured to sequentially increase the intensity of the alternating magnetic field multi-stepwise until the first period of time elapses.

3. The cancer treatment apparatus according to claim 1 or 2, wherein the magnetic field controller is configured to continuously change the intensity of the alternating magnetic field until the first period of time elapses.

4. The cancer treatment apparatus according to claim 1, 2, or 3, wherein the magnetic field controller is configured to increase an average of the intensity of the alternating magnetic field over time from the application of the alternating magnetic field to the living body until the timing to end the application.

5. The cancer treatment apparatus according to any one of claims 1 to 4, further comprising:
a state sensor configured to sense a heat generating state of the living body,
wherein the magnetic field controller is configured to adjust the intensity of the alternating magnetic field until the first period of time elapses such that the heat generating state is excluded from exceeding a previously set threshold based on the heat generating state sensed by the state sensor.

6. A method of controlling a cancer treatment apparatus including a magnetic field generator configured to generate an alternating magnetic field to be applied to a living body, the method comprising:
increasing an intensity of the alternating magnetic field generated by the magnetic field generator stepwise until a first period of time elapses since beginning application of the alternating magnetic field to the living body; and
setting the intensity of the alternating magnetic field to a previously set maximum value from elapse of the first period of time until a timing to end the application of the alternating magnetic field.
